# NEW EUROPEAN PATENT SPECIFICATION

(11) **EP 0 498 272 B2**
(45) Date of publication and mention of the opposition decision: **02.12.1998**
(45) Mention of the grant of the patent: 19.07.1995
(21) Application number: 92101364.5
(22) Date of filing: 28.01.1992
(51) Int. Cl.: A61K 7/50

(54) **Effervescent bathing preparation**
Brausendes Bademittel
Composition effervescente pour le bain

(30) Priority: 31.01.1991 US 648871
(43) Date of publication of application: 12.08.1992
(73) Proprietor: Kao Corporation, Chuo-ku Tokyo (JP)
(72) Inventor: Rau, Allen H., Cincinnati, Ohio 45249 (US)
(74) Representative: Wächtershäuser, Günter, Prof. Dr.

(56) References cited:
- EP-A- 0 333 223
- EP-A- 0 370 969
- GB-A- 292 042
- GB-A- 297 805
- GB-A- 776 293
- US-A- 4 342 743
- US-A- 4 650 667
- US-A- 5 002 758
- STN FILE SUPPLIER, Karlsruhe, DE, FILE CHEMICAL ABSTRACT, vol. 115, no. 14,abstract no. 142022a, Columbus, Ohio, US; &JP-A-3 066 610 (TSUMARA AND CO.) 22-03-1991
- G.A. NOWAK: "Die Kosmetischen Präparate", 2nd edition, pages 672-674, Verlag für chem. Industrie H. Ziolkowsky KG, Augsburg, DE
- Monograph for colloidal oatmeal of Federal register, vol. 54 no. 190, pages 40 808-40 813 (03.10.89);
- Reprint of Article in "Archives of Dermatology and Syphilology, vol. 68, pages 402-407 (Oct./53), pages 8-13;
- Article in "Archives of Dermatology and Syphilology", vol. 63, page 502 (09.04.51);
- G.A. Nowak: "Die kosmetischen Präparate", 2. Auflage, pages 673 - 674.

## Description

This invention pertains to a for treating inflammation of the skin comprising a colloidal material, and more particularly to bathing tablets having an enhanced ability of dispersing the colloidal material, while providing skin stimulation for fatigue relief by effervescence and/or carbon dioxide, and treating skin inflammation by the colloidal material.

Bathing preparations which dissolve in warm or hot bath water to effervesce by the release of carbon dioxide as a reaction product in order to stimulate the skin and provide fatigue relief are well known. GB-A-776 293 discloses a method for producing carbon dioxide containing baths, whereby water-soluble colloids are used to prevent the formation of insoluble precipitates upon addition of sodium bicarbonate. Bathing preparations containing colloids for enhancing the carbon dioxide release are known from G.A. Nowak: "Die kosmetischen Präparate", 2nd edition, pages 673 to 674. Another example is that disclosed in U.S. Patent 4,666,707. In this patent, a moisturizer is incorporated in a carbonate salt/ acid combination. When dissolved, the carbonate and acid react to release carbon dioxide. The weakly acidic pH of the resulting water keeps CO₂ in solution, prolonging and promoting the effects of CO₂ skin stimulation, synergistically enhanced by the moisturizer.

Other effervescing bath preparations are also known. A fundamental preparation is disclosed in U.S. Patent 4,650,667. A further example is disclosed in U.S. Patent 5,002,758.

This preparation employs low-cost fumaric acid. Preparations of the type described are in commercial use, and available under the trademark ACTIBATH™ as well as other names.

The treatment of minor skin irritations, and in particular, relief from itching induced by inflammation, disease, trauma and the like, through the water-mediated application of colloidal materials is well known. Prominent among various materials of this type is colloidal oatmeal, commercially available under the mark AVEENO™ from S.C. Johnson Company, as well as from other sources. Hydrophobic starches are also well known in this application, as materials which, when dispersed in water, settle on and desensitize the skin, and provide temporary relief.

In general, the colloidal material treatments for skin inflammation are effected by dispersing the colloidal material in bath water, and then bathing in the bath suspension. This method presents a number of problems.

Maximum effectiveness of the above-mentioned treatments is achieved by thorough dispersion of the colloidal material throughout the bath water. As these materials are generally in powder form, not only is this a physically awkward operation, but thorough dispersion tends to be frustrated by the tendency of these materials to sell, soften and agglomerate upon moistening.

Further, agglomerated particles of this type tend to settle relatively rapidly, according to Stokes Law, which provides a particle's settling rate in a dispersing fluid will be governed by its relative diameter and density as well as the fluid's viscosity and density.

Due to the colloidal nature of this material, it tends to persist in the tub or bathing enclosure even after draining of the water. The greater the degree of agglomeration, the more difficult it may be to remove residual material.

It is therefore an object of the present invention to provide a bathing preparation for treating inflammation of the skin which has the benefits of carbon dioxide bathing as well as the benefits of colloidal material bathing, and which dose not cause any bad influences to a tub or bathing enclosure.

Under these circumstances, the present inventors have made extensive studies, and have found that when a material releasing carbon dioxide and a colloidal material are included into a bathing tablet the bathing tablet effervesces when dissolved in warm or hot bath water, and that the effervescing activity serves to automatically disperse the colloidal material, thereby preventing the dispersed colloidal material from agglomeration, and accelerating the colloidal particles away from the bathing tablet throughout the bath water. The present inventors have also found that such bath tablet has the advantages of bathing with carbon dioxide. This invention has been achieved based upon these findings.

Namely, this invention provides a tablet for treating inflammation of the skin comprising (a) a material which will dissolve in warm and hot water to release carbon dioxide, and (b) 0.1 - 50 wt.% of a colloidal material based on the total amount of the bathing preparation.

It is preferred that the material (a) of this invention, which releases carbon dioxide when dissolved in water, be a combination of an acid and a carbonate salt which are selected to release carbon dioxide when dissolved in water, especially in warm or hot bath water. Exemplary combinations of this type are set forth in U.S. Patent 4,666,707. Examples of the carbonate salt include sodium hydrogen carbonate, sodium carbonate, sodium sesquicarbonate, potassium hydrogen carbonate, potassium carbonate, potassium sesquicarbonate, ammonium hydrogen carbonate, ammonium carbonate, and ammonium sesquicarbonate. These may be used either alone, or as a mixture of two or more.

Examples of the acid which can be combined with the carbonate salt includes formic acid, straight-chain aliphatic acids such as acetic acid, propanoic acid, butyric acid and valeric acid; dicarboxylic acids such as oxalic acid, malonic acid, succinic acid, glutaric acid, adipic acid, pimelic acid, fumaric acid, maleic acid, phthalic acid, isophthalic acid and terephthalic acid; acidic amino acids such as glutamic acid and aspartic acid; hydroxy acids such as glycolic acid, lactic acid, hydroxy acrylic acid, α-hydroxy butyric acid, glyceric acid, tartronic acid, maleic acid, tartaric acid, malic acid, hydroxy benzoic acid, citric acid, salicylic acid, gallic acid, mandelic acid, tropic acid, ascorbic acid and gluconic acid; cinnamic acid, benzoic acid, phenylacetic acid, nicotinic acid, kainic acid, sorbic acid, pyrrolidone carboxylic acid, trimellitic acid, benzene sulfonic acid and toluene sulfonic acid; and acidic salts of these organic acids. Inorganic acids may also be used, including phosphoric acid, potassium dihydrogen phosphate, sodium dihydrogen phosphate, sodium sulfite, potassium sulfite, sodium pyrosulfite, potassium pyrosulfite, acidic sodium hexametaphosphate, acidic potassium hexametaphosphate, acidic sodium pyrophosphate, acid potassium pyrophosphate and sulfamic acid. Preferred examples of carbonate/acid combinations include sodium carbonate and/or sodium bicarbonate together with succinic and/or fumaric acid.

Although any blending ratio of carbonate salt and acid can be used in so far as the intended effects of this invention are obtained, it is preferred that the acid be present in amounts of 10 - 350 wt.%, based on the carbonate salt.

As detailed in U.S. Patent 5,002,758 when using fumaric acid, it is advantageous to incorporate either carboxymethyl cellulose (0.1 - 20 wt.% based on fumaric acid content) or polyethylene glycol (0.2 - 20 wt.% based on fumaric acid content) together with 0.02 - 0.1 wt.% based on fumaric acid content of a nonionic surfactant having a hydrophile-lipophile balance (HLB) of 7 or more.

Examples of the nonionic surfactant having a hydrophile-lipophile balance of 7 or more include sucrose fatty acid ester, polyglyceryl fatty acid ester, propylene glycol fatty acid ester, sorbitan fatty acid ester, polyoxyethylene sorbitan fatty acid ester, polyoxyethylene glycol fatty acid ester.

Examples of the colloidal material, the component (b) of this invention, include a powdery material which forms colloid when dispersed in water. They are colloidal oatmeal, flours derived from corn, wheat, soy, rice, barley or other grains; cornmeal, almond meals and meals prepared from similar sources; hydrophobic or water-insoluble starch obtained from corn, wheat, rice, potato, or other grains, water-insoluble gums, cellulose, as well as mixtures of these materials. Colloidal oatmeal constitutes a preferred colloidal material for use in the claimed invention. Depending on the materials selected and the effect desired, the amount of colloidal material incorporated may vary from 0.1 - 50 wt.%, preferably 1 - 30 wt.%, of the total composition of the bath preparation. With regard to colloidal oatmeal, documents submitted to the Food and Drug Administration by the manufacturer of AVEENO™ indicate that a final bath concentration of 0.007 - 10 wt.% colloidal oatmeal is sufficient to provide the intended benefit. The tablets may be formulated to provide this concentration in a single tablet, or using a plurality of tablets for each bath. If the formulation employs 20 wt.% colloidal oatmeal in a 50 gram tablet, the colloidal material obtained from one tablet will be present at a final concentration of 0.01 wt.%, assuming 25 gallons of water are present in the bath, providing 10 grams of colloidal oatmeal in 84.625 liters. It should be noted that at these types of concentrations, even if 40 gallons of water are used for the bath, the resulting concentration will be 0.007 wt.%, again, within the indicated guidelines.

It is to be noted that, to achieve the aforementioned objects of this invention, it is necessary that the component (a) and component (b) are included in the same preparation. Not only is tablet form more convenient than current methods of delivering colloidal material to bath water, a simple mixture of raw materials added to bath water will result in rapid, uncontrolled gas generation without effective dispersion of the colloidal, particulate material. By using a tablet form, gas is generated in a controlled release to accelerate colloidal particles away and up from the bottom of the bath, improving dispersion. To this end, tableting/stabilizing materials may be used in the preparation of the tablet. Among these materials are polyethylene glycol, which, as noted above, also serves to suppress fumaric acid foaming. Crystalline sorbitol and magnesium oxide have also been demonstrated to be effective binder/stabilizer components. Other possible binders and stabilizers include sodium aluminate, microcrystalline cellulose, sodium alginate, polyvinyl alcohol, polyvinyl pyrrolidone, lactose, dextran, dextrose monohydrate, starch, dibasic calcium phosphate dihydrate, sucrose, sugar, calcium sulfate dihydrate, carboxymethyl starch, polyacrylate copolymers and polymethacrylate. Reference is made to Lieberman et al, ed., Pharmaceutical Dosage Forms: Tablets, Volume 1, Chapter 2, "Tablet Formulation and Design", for further information regarding the preparation of stabilized tablets.

Other additives which may be included in amounts which will not interfere with effervescing activity or be irritating to the skin include perfumes, moisturizers, colorants, emollients, skin protectants, humectants, softening agents, sunscreens and the like. To improve tableting, flow improvers may be included in amounts of 0.1 - 5.0 wt.%, including such materials as calcium silicate, fumed silica, precipitated silica, hydrated silica, aluminum starch octenyl succinate, as well as other materials referred to in Lieberman, supra.

If the ratio of carbonate salt and acid is chosen so that the final product pH (0.01% solution) is weakly acid (pH 4 - 7), the benefits of carbon dioxide bathing, namely the effect of improving the circulation of the blood by carbon dioxide present as dissolved, discussed in U.S. Patent 4,666,707, can be simultaneously obtained, along with the benefits of colloidal oatmeal. At alkaline pH, carbon dioxide will be generated to disperse the colloidal particles, but will not remain dissolved in the water. Thus, under alkaline conditions, the invention herein can be used, but only the benefits of dispersion of colloidal materials will be apparent.

The effervescing activity by the component (a) of the bath tablet according to this invention serves to enhance dispersion of the colloidal material by preventing agglomeration of the material while simultaneously accelerating the particles away from the tablet throughout bath water. By placing the colloidal material in close proximity to the source of effervescence, the colloidal material may be "auto-dispersed" throughout the bath water, eliminating the need to disperse the material by introducing it into a stream of running water by hand sprinkling, coupled with subsequent stirring of water. After bathing, the colloidal material having agglomerated to a reduced degree, if at all, the material is easier to drain from the tub or bathing enclosure. The bathing preparation according to this invention also gives the benefits of carbon dioxide bathing while giving an excellent treatment effect by the colloidal material for skin inflammation.

The present invention will now be described in detail with reference to a preferred embodiment. It, however, should not be construed as limiting the scope of the present invention thereto.

### Example 1:

An example of the claimed invention was prepared by tableting a combination of sodium bicarbonate 18.460 wt.%, sodium carbonate 9.100 wt.%, succinic acid 22.448 wt.%, fumaric acid 9.638 wt.% and colloidal oatmeal, having a particle size meeting the definition proposed by S.C. Johnson to the FDA of less than 3 wt.% greater than 150 microns, and less than 20 wt.% greater than 75 microns, 20 wt.%. Additionally, for the formation of tablets, polyethylene glycol (molecular weight 6000) was added, as binders and stabilizers, in amount of 2.150 wt.%, crystalline sorbitol in an amount of 18.180 wt.% and magnesium oxide in amount of 0.20 wt.%. In light of the presence of fumaric acid, an additional 0.004 wt.% sucrose stearate was added, to complete the composition of the invention.

This example of the invention, as well as comparison examples were placed in separate hot bath water (150 liters, 40°C). The water was stirred with a pole (ten revolutions, slow speed). After 4 minutes the water was passed through a sieve (250 micron openings). The residue was dried at 105°C for 5 hours. The amount of material remaining is set forth in table 1. The pH of the bath water, in which the bathing preparation of this example had been placed, was 5.05, and the colloidal material of this example dispersed thoroughly, and no residual material existed.

**TABLE 1**

| Sample No. | Material | Grams Remaining |
|---|---|---|
| 1 | Example of the Invention (10 g colloidal oatmeal) | 0.0 |
| 2 | Colloidal Oatmeal (10g) | 2.0 |
| 3 | Colloidal Oatmeal (10 g) and Sodium Bicarbonate (9.3 g) | 0.7 |
| 4 | Colloidal Oatmeal (10 g) and Succinic Acid (11.9 g) | 1.4 |
| 5 | AVEENO™ (S.C. Johnson, 20 g) | 1.2 |

## Claims (Claims for the following Contracting State(s): DE, FR, GB)

1. A tablet for treating inflammation of the skin comprising:
(a) a material which will dissolve in warm and hot water to release carbon dioxide; and
(b) 0.1 to 50 % by weight of a colloidal material, based on the total amount.

2. A tablet according to Claim 1, wherein the material (a) is a combination of a carbonate salt and an acid.

3. A tablet according to Claim 1, wherein the colloidal material (b) is selected from the group comprising colloidal oatmeal, flour derived from corn, wheat, soy, rice, barley or other grains, cornmeal, almond meal, hydrophobic or water-insoluble starch obtained from corn, wheat, rice, potato or other grains, water-insoluble gums and cellulose.

## Claims (Claims for the following Contracting State(s): ES)

1. Process for obtaining a tablet for treating inflammation of the skin, characterized by comprising the suitable mixing of the following components:
(a) a material which will dissolve in warm and hot water to release carbon dioxide; and
(b) 0.1 to 50% by weight of a colloidal material, based on the total amount;
and to formulate the thus obtained mixture in the form of tablets.

2. A process according to Claim 1, wherein the material (a) is a combination of a carbonate salt and an acid.

3. A process according to Claim 1, wherein the colloidal material (b) is selected from the group comprising colloidal oatmeal, flour derived from corn, wheat, soy, rice, barley or other grains, cornmeal, almond meal, hydrophobic or water-insoluble starch obtained from corn, wheat, rice, potato or other grains, water-insoluble gums and cellulose.

## Patentansprüche (Patentansprüche für folgende(n) Vertragsstaat(en): DE, FR, GB)

1. Tablettezur Behandlung von Hautentzündungen, umfassend:
(a) ein Material, das sich in warmem und heißem Wasser auflöst, um Kohlendioxid abzugeben; und
(b) 0,1 bis 50 Gew.-% eines kolloidalen Materials, bezogen auf die Gesamtmenge.

2. Tablettegemäß Anspruch 1, wobei das Material (a) eine Kombination eines Carbonatsalzes und einer Säure ist.

3. Tablettegemäß Anspruch 1, wobei das kolloidale Material (b) ausgewählt wird aus der Gruppe, umfassend kolloidale Haferflocken, Mehl, erhalten aus Mais, Weizen, Soja, Reis, Gerste oder anderem Getreide, Maisschrotmehl, Mandelschrot, hydrophobe oder wasserunlösliche Stärke, erhalten aus Mais, Weizen, Reis, Kartoffel oder anderem Getreide, wasserunlöslichen Gummis und Cellulose.

## Patentansprüche (Patentansprüche für folgende(n) Vertragsstaat(en): ES)

1. Verfahren zur Herstellung eines Mittels zur Behandlung von Hautentzündungen, gekennzeichnet dadurch, daß es das geeignete Mischen der folgenden Bestandteile umfaßt:
(a) ein Material, das sich in warmem und heißem Wasser unter Kohlendioxidabgabe auflöst; und
(b) 0,1 bis 50 Gew.-% eines kolloidalen Materials, bezogen auf die Gesamtmenge;
und die Formulierung des so erhaltenen Gemisches in Form von Tabletten,

2. Verfahren gemäß Anspruch 1, wobei das Material (a) eine Kombination aus Carbonatsalz und einer Säure ist.

3. Verfahren gemäß Anspruch 1, wobei das kolloidale Material (b) ausgewählt wird aus der Gruppe, umfassend kolloidale Haferflocken, Mehl, erhalten aus Mais, Weizen, Soja, Reis, Gerste oder anderem Getreide, Maisschrotmehl, Mandelschrot, hydrophobe oder wasserunlösliche Stärke, erhalten aus Mais, Weizen, Reis, Kartoffel oder anderem Getreide, wasserunlöslichen Gummis und Cellulose.

## Revendications (Revendications pour l'(les) Etat(s) contractant(s) suivant(s): DE, FR, GB)

1. Comprimé pour le traitement de l'inflammation de la peau, comprenant :
(a) une matière qui se dissout dans l'eau chaude et très chaude, pour libérer du dioxyde de carbone; et
(b) 0,1 à 50% en poids d'une matière colloïdale, calculés sur la quantité totale.

2. Comprimé selon la revendication 1, dans lequel la matière (a) est une combinaison d'un sel carbonate et d'un acide.

3. Comprimé selon la revendication 1, dans lequel la matière colloïdale (b) est choisie dans le groupe comprenant la farine colloïdale d'avoine, la farine dérivée de maïs, de blé, de soja, de riz, d'orge ou d'autres céréales, la farine de mais, la farine d'amandes, l'amidon hydrophobe ou insoluble dans l'eau, obtenu à partir de mais, de blé, de riz, de pommes de terre ou d'autres céréales, les gommes insolubles dans l'eau et la cellulose.

## Revendications (Revendications pour l'(les) Etat(s) contractant(s) suivant(s): ES)

1. Procédé d'obtention d'un comprimé pour le traitement de l'inflammation de la peau, caractérisé en ce qu'il comprend le mélange approprié des composants suivants :
(a) une matière qui se dissout dans l'eau chaude et très chaude, pour libérer du dioxyde de carbone; et
(b) 0,1 à 50% en poids d'une matière colloïdale, calculés sur la quantité totale,
et la formulation du mélange ainsi obtenu sous la forme de comprimés.

2. Procédé selon la revendication 1, dans lequel la matière (a) est une combinaison d'un sel carbonate et d'un acide.

3. Procédé selon la revendication 1, dans lequel la matière colloïdale (b) est choisie dans le groupe comprenant la farine colloïdale d'avoine, la farine dérivée de maïs, de blé, de soja, de riz, d'orge ou d'autres céréales, la farine de maïs, la farine d'amandes, l'amidon hydrophobe ou insoluble dans l'eau, obtenu à partir de maïs, de blé, de riz, de pommes de terre ou d'autres céréales, les gommes insolubles dans l'eau et la cellulose.
